# EUROPÄISCHE PATENTSCHRIFT

(11) **EP 0 924 198 B1**
(45) Veröffentlichungstag und Bekanntmachung des Hinweises auf die Patenterteilung: **23.01.2002**
(21) Anmeldenummer: 98115661.5
(22) Anmeldetag: 20.08.1998
(51) Int. Cl.: C07C 323/12, C07C 319/06, C07C 319/12, A23L 1/226, A61K 7/46

(54) **3-Mercapto-2-alkyl-alkan-1-ole und ihre Anwendung als Duft- und Aromastoffe**
3-Mercapto-2-alkyl-alkan-1-ols and their use as perfuming and flavouring agents
3-Mercapto-2-alkyl-alkan-ols et leur utilisation en tant qu'agents parfumants et aromatisants

(30) Priorität: 19.12.1997 DE 19756789
(43) Veröffentlichungstag der Anmeldung: 23.06.1999
(73) Patentinhaber: Dragoco Gerberding & Co Aktiengesellschaft, 37603 Holzminden (DE)
(72) Erfinder: Widder, Sabine, Dr, 37603 Holzminden (DE); Dittner, Thomas, 37619 Kirchbrak (DE); Sabater-Lüntzel, Christopher, Dr, 37620 Halle (DE); Pickenhagen, Wilhelm, Dr, 37671 Höxter (DE); Vollhardt, Jürgen, Dr, 37603 Holzminden (DE)
(74) Vertreter: Eisenführ, Speiser & Partner

(56) Entgegenhaltungen:
- DE-A- 2 316 456
- DE-A- 2 338 680
- US-A- 3 978 240
- US-A- 4 070 308

## Beschreibung

Die Erfindung betrifft einen neuen Duft- und Aromastoff sowie ein Verfahren zur Herstellung dieses neuen Stoffes.

Lebensmittel werden in der heutigen Zeit regelmäßig aromatisiert. Die Mehrheit der Konsumenten in den modernen Industriegesellschaften erwartet nämlich eine breite Palette schmackhafter Lebensmittel zu erschwinglichen Preisen. Die Schmackhaftigkeit von Lebensmitteln ist von großer Bedeutung, da sie in der Regel eine gute Bekömmlichkeit bewirkt. Die Aromenindustrie stellt bereits eine Vielzahl von Aromen zur Verfügung, um Lebensmittel einer breiten Bevölkerungsschicht verfügbar und schmackhaft zu machen.

Aromastoffe werden eingesetzt, um (a) Lebensmittelprodukten ohne Eigengeschmack eine Geschmacksnote zu verleihen oder um (b) Aromaverluste auszugleichen, die beispielsweise im Rahmen des Herstellungsprozesses eines Lebensmittels auftreten.

Aus der DE-A 2 316 456 sind bestimmte Mercaptoalkohole und Ester derselben bekannt, die einen sehr durchdringenden Geruch aufweisen, wenn sie in hohen Konzentrationen gerochen werden. In verdünnter Form und unter passend gewählten Bedingungen entwickeln sie bestimmte angenehme Geruchs- und Aromanuancen.

Aus der DE-A 2 338 680 sind bestimmte aliphatische oder cycloaliphatische Mercaptoderivate bekannt, welche unter anderem organoleptische Eigenschaften entwickeln können, die typisch sind für bestimmte exotische Früchte.

Der vorliegenden Erfindung lag die Aufgabe zugrunde, einen neuen Stoff zur Aromatisierung von Lebensmitteln (nachfolgend auch kurz "Aromastoff" genannt) anzugeben.

Erfindungsgemäß werden die 3-Mercapto-2-alkyl-alkan-1-ole der allgemeinen Formel A 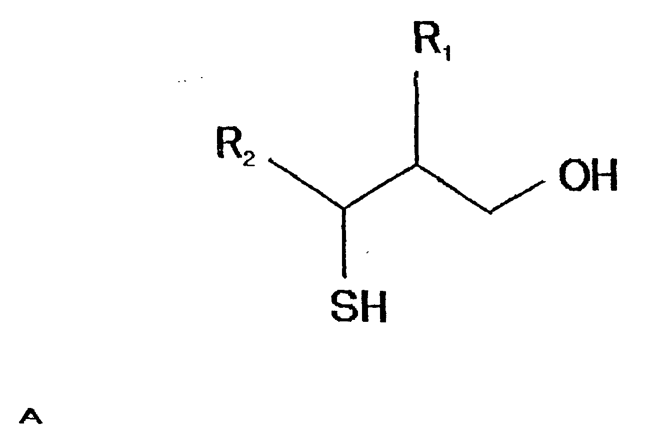 mit
R₁ = CH₃, C₂H₅, C₃H₇ (n, iso) oder C₄H₉ (n, iso, tert.) und
R₂ = CH₃, C₂H₅, C₃H₇ (n, iso) oder C₄H₉ (n, iso, tert.)
als Aromastoff angegeben. überraschenderweise hat sich nämlich gezeigt, daß diese neu synthetisierten Verbindungen hervorragend dazu geeignet sind, Lebensmitteln (insbesondere Fleischbrühen und anderen Fleischspeisen) eine interessante geruchliche und geschmackliche Note zu verleihen. Auf Grund der sehr hohen geruchlichen und geschmacklichen Intensität der erfindungsgemäßen Verbindungen können sie in großer Verdünnung eingesetzt werden; die genauen Konzentrationen oder Mengen zur Aromatisierung eines Lebensmittels wird der Fachmann auf übliche Weise an die jeweiligen Wünsche und Bedürfnisse des Einzelfalls anpassen.

Die erfindungsgemäßen Verbindungen sind überdies auch zur Verwendung als Duftstoff geeignet, und zwar insbesondere als Duftstoff in der Parfümindustrie; sie haben generell einen extern niedrigen Geruchsschwellenwert, was sich als vorteilhaft erweist, da bereits geringe Mengen einer erfindungsgemäßen Verbindung zur Erzeugung eines erwünschten Geruches ausreichen.

Den erfindungsgemäßen Verbindungen gemeinsam sind folgende strukturelle Merkmale, die überraschenderweise nur bei gleichzeitiger Anwesenheit in einem Molekül die erwünschten geruchlichen und geschmacklichen Eigenschaften erzeugen:
- eine Alkoholfunktion am C-1
- eine Mercapto-Gruppe (SH-Gruppe) am C-3
- ein tertiäres C-2.

Die Erfindung betrifft neben den erfindungsgemäßen Verbindungen auch Verfahren zu ihrer Herstellung.

Bei einem erfindungsgemäßen Verfahren zur Herstellung eines 3-Mercapto-2-alkyl-alkanols der Formel A mit R₁ = CH₃, C₂H₅, C₃H₇ (n, iso) oder C₄H₉ (n, iso, tert.) und R₂ = CH₃, C₂H₅, C₃H₇ (n, iso) oder C₄H₉ (n, iso, tert.) wird das korrespondierende 3-Acetylthio-2-alkyl-alkanal mit einem Reduktionsmittel wie Lithiumaluminiumhydrid oder Diisobutylaluminiumhydrid umgesetzt.

Dieses Verfahren ist insbesondere zur Herstellung des 3-Mercapto-2-methyl-pentan-1-ol (Verbindung der Formel A mit R₁ = CH₃ und R₂ = C₂H₅) geeignet. Es wird dabei 3-Acetylthio-2-methyl-pentanal mit einem Reduktionsmittel wie Lithiumaluminiumhydrid oder Diisobutylaluminiumhydrid umgesetzt.

Bei einem alternativen Verfahren zur Herstellung eines 3-Mercapto-2-Alkyl-Alkanols der Formel A mit R₁ = CH₃, C₂H₅, C₃H₇ (n, iso) oder C₄H₉ (n, iso, tert.) und R₂ = CH₃, C₂H₅, C₃H₇ (n, iso) oder C₄H₉ (n, iso, tert.) wird das korrespondierende 3-Mercapto-2-Alkyl-Alkanal mit einem Reduktionsmittel wie Natriumborhydrid, Lithiumaluminiumhydrid oder Diisobutylaluminiumhydrid umgesetzt.

Auch dieses Verfahren ist insbesondere zur Herstellung des 3-Mercapto-2-methyl-pentan-1-ol geeignet.

Die Enantiomeren des 3-Mercapto-2-methyl-pentan-1-ol und der anderen erfindungsgemäßen Alkanole lassen sich im Wege einer enantiomerenreinen Synthese erhalten, wie nachfolgend noch näher erläutert wird.

Entsprechend dem bevorzugten Einsatzgebiet der erfindungsgemäßen Verbindungen betrifft die Erfindung auch eine Duft- oder Aromastofformulierung, die zumindest eine erfindungsgemäße Verbindung umfaßt.

Und schließlich betrifft die Erfindung noch aromatisierte Lebensmittel mit einem Anteil an einer oder mehreren erfindungsgemäßen Verbindungen sowie die Verwendung der erfindungsgemäßen Verbindungen als Duft- oder Aromastoff.

Die erfindungsgemäßen Alkanole sind sensorisch besonders interessant. Anstelle ihres direkten Einsatzes als Duft- oder Aromastoff ist es aber manchmal erwünscht, chemische Derivate einzusetzen, die sich leicht in das jeweilige Alkanol umwandeln lassen. Die Derivate können dabei selbst sensorisch interessant sein, müssen es aber nicht. Insbesondere ist es möglich, einem Lebensmittel statt eines erfindungsgemäßen Alkanols den korrespondierenden Ester zuzusetzen (z.B. das entsprechende Acetat, Propionat, Butyrat etc.). Dies ist insbesondere dann sinnvoll, wenn bei der Weiterbehandlung des Lebensmittels eine Hydrolyse des Esters zum sensorisch aktiven Alkanol stattfindet.

Ein typisches Beispiel hierfür ist die Behandlung von üblicherweise zu kochenden, zu bratenden oder zu grillenden Fleischspeisen mit dem Ester (z.B. dem Acetat) eines erfindungsgemäßen Alkanols. Beim Koch-, Brat- oder Grillvorgang findet nämlich regelmäßig eine (zumindest partielle) Hydrolyse des Esters zum erfindungsgemäßen Alkanol statt, welcher dann die eigentliche aromatisierende Funktion übernimmt. Gemäß einem weiteren typischen Beispiel erfolgt die Erzeugung des Alkanols aus dem korrespondierenden Ester bereits industriell, und zwar nach den üblichen Verfahren der industriellen Behandlung von Lebensmitteln mit Reaktionsaromen.

Gegenstand der Erfindung ist dementsprechend auch ein Verfahren zur Aromatisierung eines Lebensmittels mit einem erfindungsgemäßen Alkanol, wobei
- das Lebensmittel mit einem Ester des Alkanols versetzt
- und das mit dem Ester versetzte Lebensmittel einer Behandlung unterzogen wird, bei der der Ester zumindest partiell in das Alkanol überführt wird.

Der Begriff "Ester des Alkanols" umfaßt dabei auch die 3-Acylthioester eines erfindungsgemäßen Alkan-1-ols. Diese lassen sich beispielsweise durch Veresterung des erfindungsgemäßen Alkan-1-ols mit einem Carbonsäurechlorid (z.B. Essigsäurechlorid oder Propionsäurechlorid) erhalten.

Nachfolgend wird die Erfindung anhand von Beispielen näher erläutert:

Die in den nachstehenden Beispielen hinter den Reagenzien aufgeführten Klammerterme, [623-36-9] usw., sind die Chemical-Abstracts-Nummern der jeweiligen Reagenzien.

Die zunächst folgenden Beispiele 1 und 2 bilden zusammen eine Herstellvorschrift für die erfindungsgemäße Gruppe der 3-Mercapto-2-alkyl-alkan-1-ole am Beispiel des 3-Mercapto-2-methyl-pentan-1-ol, wobei in einer ersten Stufe (Beispiel 1) 3-Acetylthio-2-methyl-pentanal hergestellt wird.

### Beispiel 1: Herstellung von 3-Acetylthio-2-methyl-pentanal

Reagenzien:
2-Methyl-2-pentenal (623-36-9)
Thioessigsäure (507-09-5)
Piperidin (110-89-4)

In einer 50ml-Rührapparatur mit Innenthermometer wird unter Stickstoffatmosphäre bei 10°C zu 13,47g 2-Methyl-2-pentenal 0,14g Piperidin gegeben. Danach tropft man unter Rühren langsam 15,73g Thioessigsäure bei 10°C (Eis/Wasser-Kühlung) dazu. Nach der Zugabe läßt man die Reaktion 18h bei Raumtemperatur weiterrühren. Anschließend verdünnt man das Reaktionsgemisch mit 100ml Diethylether. Die organische Phase wird nacheinander einmal mit 20ml 1N Salzsäure und zweimal mit je 20ml gesättigter Natriumhydrogencarbonatlösung gewaschen. Nach Trocknung der organischen Phase über Natriumsulfat, wird das Lösungsmittel am Rotationsverdampfer abgezogen. Man erhält 16,8g einer fast farblosen Flüssigkeit. GC-MS-Analyse des Rohprodukts zeigt eine 82%-ige Reinheit des gewünschten Produkts an (Diastereomerenverhältnis 49:51). Das empfindliche Rohprodukt wird ohne weitere Aufreinigung in der Folgereaktion gemäß Beispiel 2 eingesetzt.

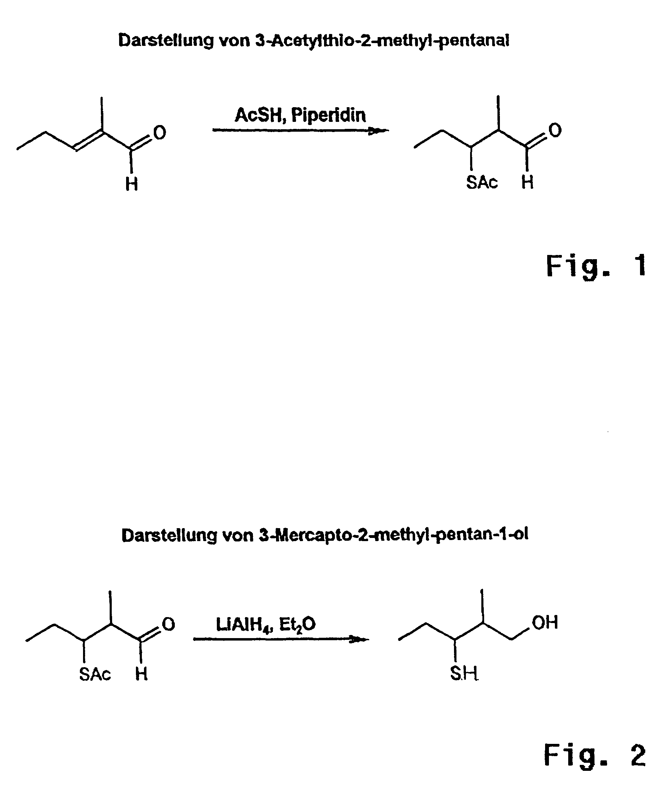

Die Herstellung von 3-Acetylthio-2-methyl-pentanal gemäß Beispiel 1 ist in der beigefügten Figur 1 schematisch dargestellt.

### Beispiel 2: Herstellung von 3-Mercapto-2-methyl-pentan-1-ol

Reagenzien:
Lithiumaluminiumhydrid
3-Acetylthio-2-methyl-pentanal

In eine trockene 500ml Rührapparatur mit Innenthermometer gibt man unter Stickstoffatmosphäre zu 3,6g Lithiumaluminiumhydrid 100ml trockenen Diethylether und kühlt dann auf -5°C ab (Eis(Eis/Kochsalzmischung). Unter Rühren tropft man nun langsam 15g 3-Acetylthio-2-methyl-pentanal (Rohprodukt aus der ersten Stufe) zu, so daß die Innentemperatur 5°C nicht übersteigt. Nach dem Zutropfen läßt man noch eine halbe Stunde bei Raumtemperatur nachrühren. Man kühlt nun wieder auf 0°C ab und hydrolysiert sehr vorsichtig zunächst mit gesättigter Ammoniumchloridlösung, dann mit 2N Salzsäure. Man trennt die organische Phase ab und extrahiert die wäßrige Phase einmal mit 100ml Diethylether. Die vereinigten organischen Phasen werden zweimal mit je 20ml gesättigter Natriumhydrogencarbonatlösung gewaschen. Nach Trocknung der organischen Phase über Natriumsulfat wird das Lösungsmittel am Rotationsverdampfer abgezogen. Das Rohprodukt wird im Vakuum destilliert (Siedebereich 46-49°C/1-2mbar). Man erhält so 5,6g (30% Ausbeute über die zwei Stufen gemäß Beispiel 1 und 2) 3-Mercapto-2-methyl-pentan-1-ol (Diastereomerengemisch) als klare Flüssigkeit von intensivem Geruch.

Die Herstellung von 3-Mercapto-2-methyl-pentan-1-ol gemäß Beispiel 2 ist in der beigefügten Figur 2 schematisch dargestellt.

Spektroskopische Daten des Diastereomerengemisches von 3-Mercapto-2-methyl-pentan-1-ol (Verbindungen A und B, Verhältnis 49:51):
^{**1**}**H-NMR-Spektrum** (300 MHz, d₆-Benzol, 300 K, TMS als Standard): δ = 0.73 (d, 7 Hz, 3 H, 2-Me, B), 0.88 (d, 7 Hz, 3 H, 2-Me, A), 0.92 (t, 7 Hz, 3 H, 5-H, A od. B), 0.925 (d, 8,5 Hz, 1 H, SH, B), 0.93 (t, 7 Hz, 3 H, 5-H, B od. A), 1.15 (d, 8 Hz, 1 H, SH, A), 1.26 (br.m, 1 H, 4-Hₐ, A), 1.38 (m, 2 H, 4-H, B), 1.55 (dqd, 4 Hz, 7 Hz, 15 Hz, 4-H_{b}A), 1.68 (br. sept., ca. 7 Hz, 1 H, 2-H, A), 1.74 (br. m, 1 H, 2-H, B), 2.26 (br. s, 2 H, OH), 2.62 (dddd, 4 Hz, 5,5 Hz, 8 Hz, 10 Hz, 1 H, 3-H, A), 2.89 (ddt, 4 Hz, 6 Hz, 9 Hz, 1 H, 3-H, B), 3.35 (dd, 6 Hz, 10 Hz, 1 H, 1-Hₐ, B), 3.43 (dd, 6 Hz, 10,5 Hz, 1 H, 1-Hₐ, A), 3.47 (dd, 7 Hz, 10,5 Hz, 1 H, 1-H_{b}, A), 3.52 (dd, 8 Hz, 10,5 Hz, 1 H, 1-H_{b}, B).
^{**13**}**C-NMR-Spektrum** (75 MHz, d₆-Benzol, 300 K, TMS als Standard): δ = 10.5 (1°, 2-Me, B), 12.2; 12.6 (1°, C-5, A u.B), 14.4 (1°, 2-Me, A), 28.3 (2°, C-4, A), 30.6 (2°, C-4, B), 40.3 (3°, C-2, B), 42.2 (3°, C-2, A), 44.4 (3°, C-3, B), 45.7 (3°, C-3, A), 65.3 (2°, C-1, A), 65.9 (2°, C-1, B).
(1° = prim., 2° sek., 3° = tert. C-Atom)
**FTIR:** Verbindung A: 3669, 3582, 2971, 2939, 2891, 1464, 1385, 1033; Verbindung B: 3669, 3583, 2972, 2939, 2889, 1465, 1385, 1035
**MS** (EI, 70 eV): Verbindung A: 134 (M⁺, 21), 100 (42), 83 (18), 75 (51), 74 (100), 71 (60), 55 (48), 47 (30), 45 (27), 41 (96), 31 (33); Verbindung B: 134 (M⁺, 21), 100 (42), 83 (22), 75 (52), 74 (100), 71 (60), 55 (50), 47 (31), 45 (31), 41 (97), 31 (34).

Anstelle des in der zweiten Herstellungs-Stufe gemäß Beispiel 2 eingesetzten Lithiumaluminiumhydrids kann Diisobutylaluminiumhydrid oder ein anderes geeignetes Reduktionsmittel Verwendung finden. Die Aufarbeitungsprozeduren sind dann gegebenenfalls auf übliche Weise an das eingesetzte Reduktionsmittel anzupassen.

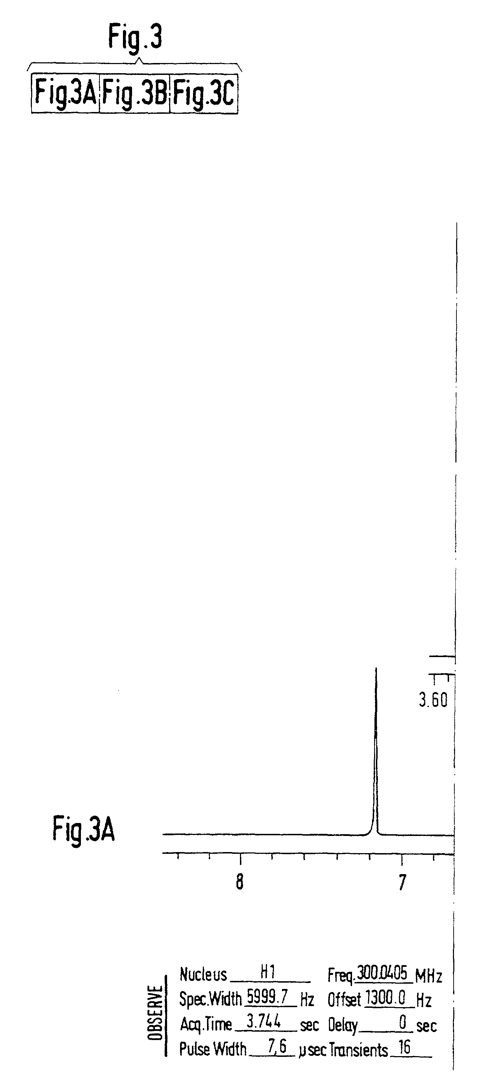

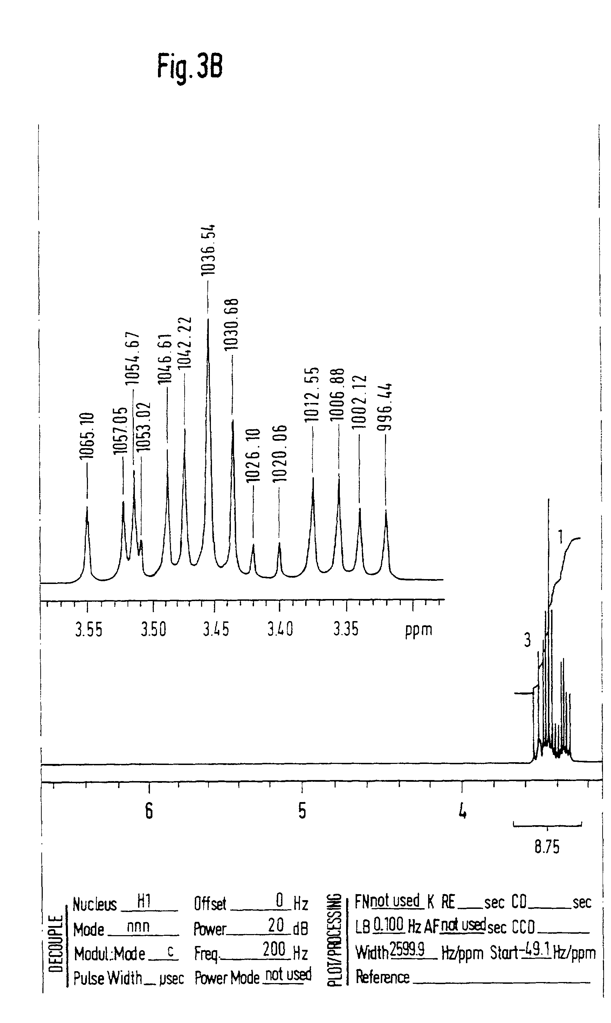

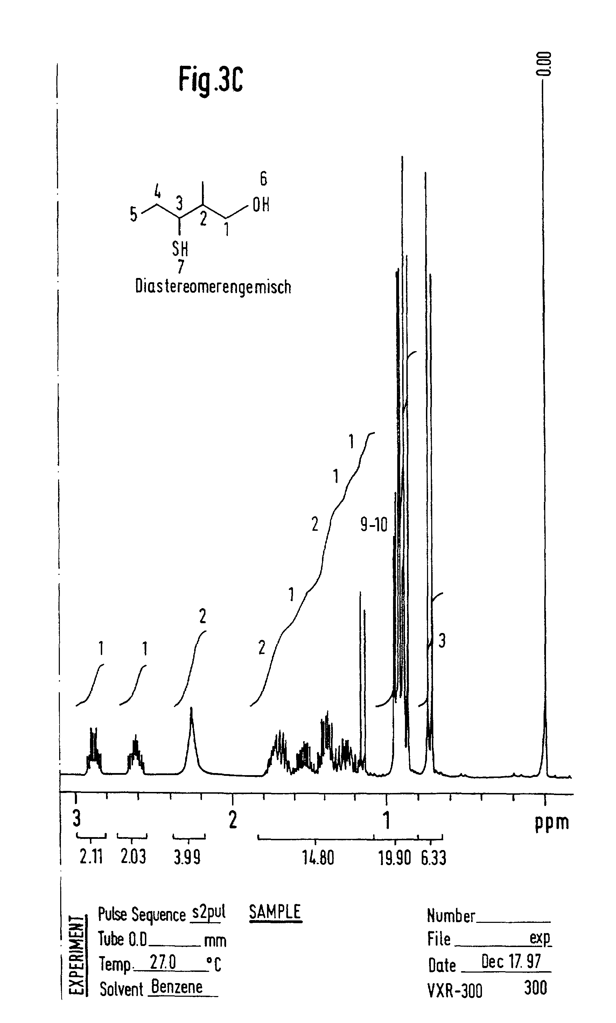

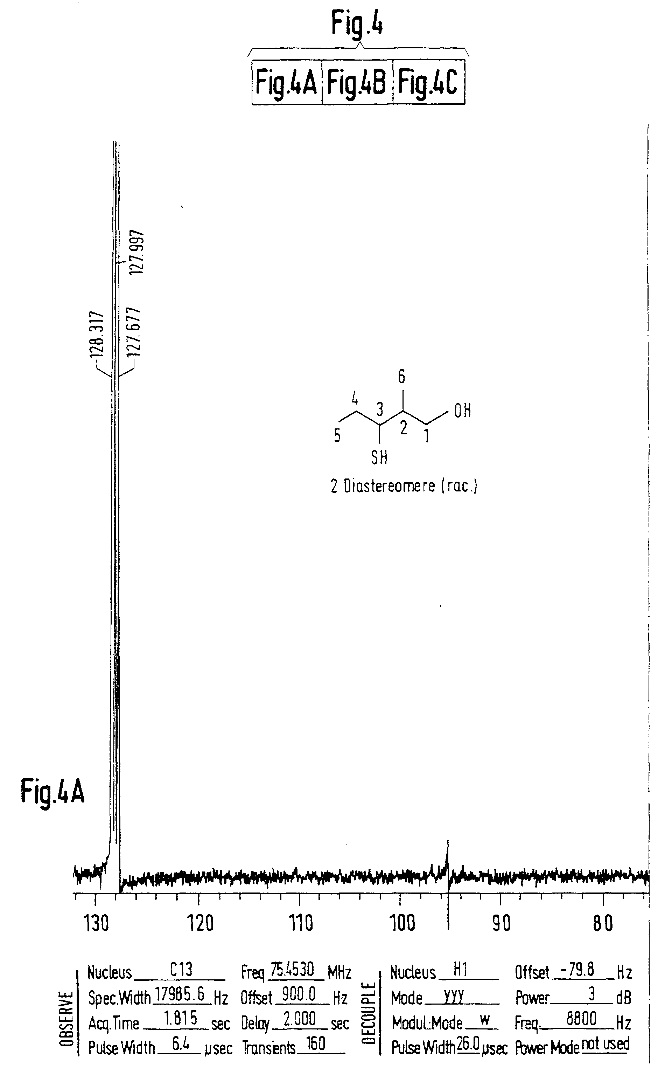

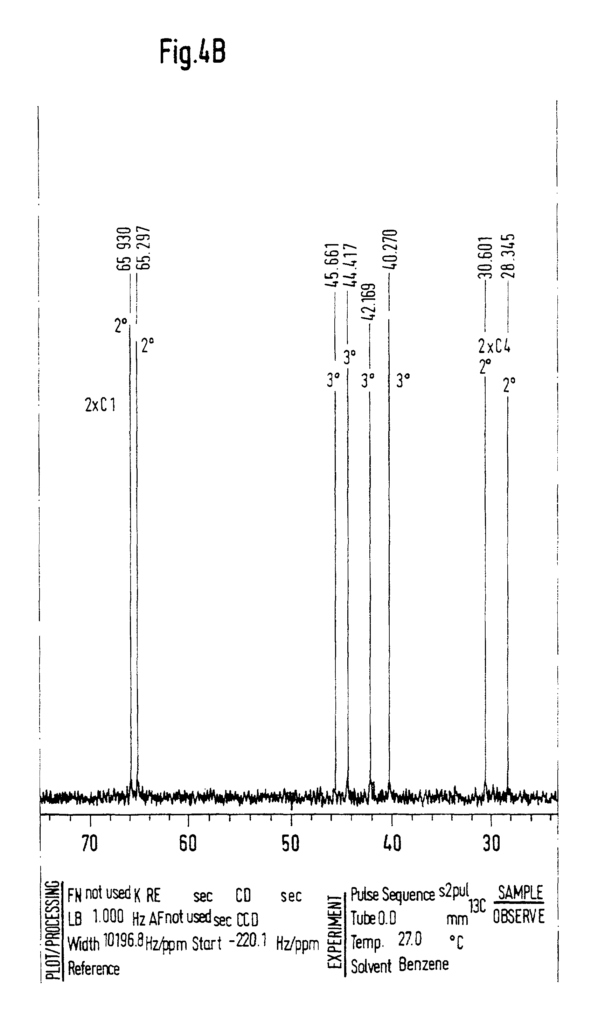

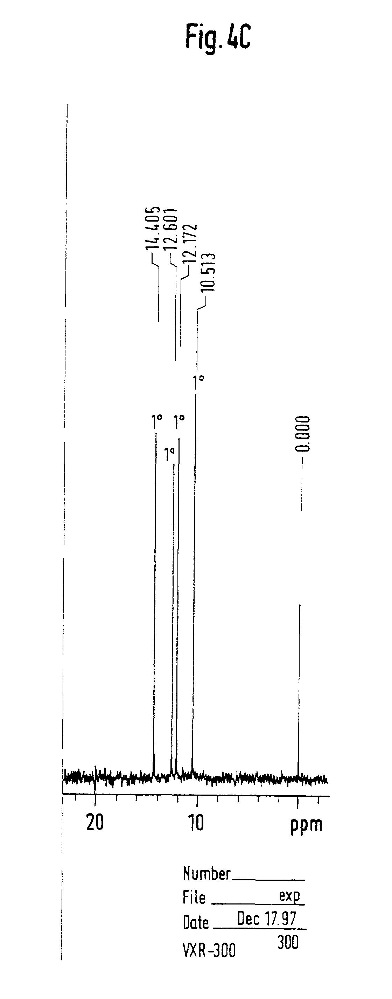

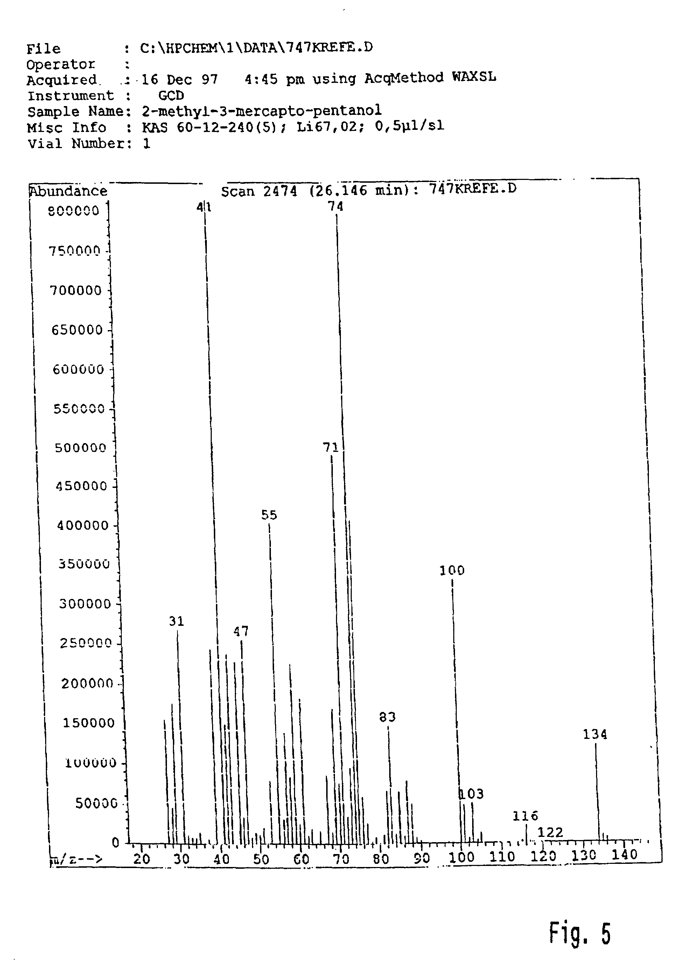

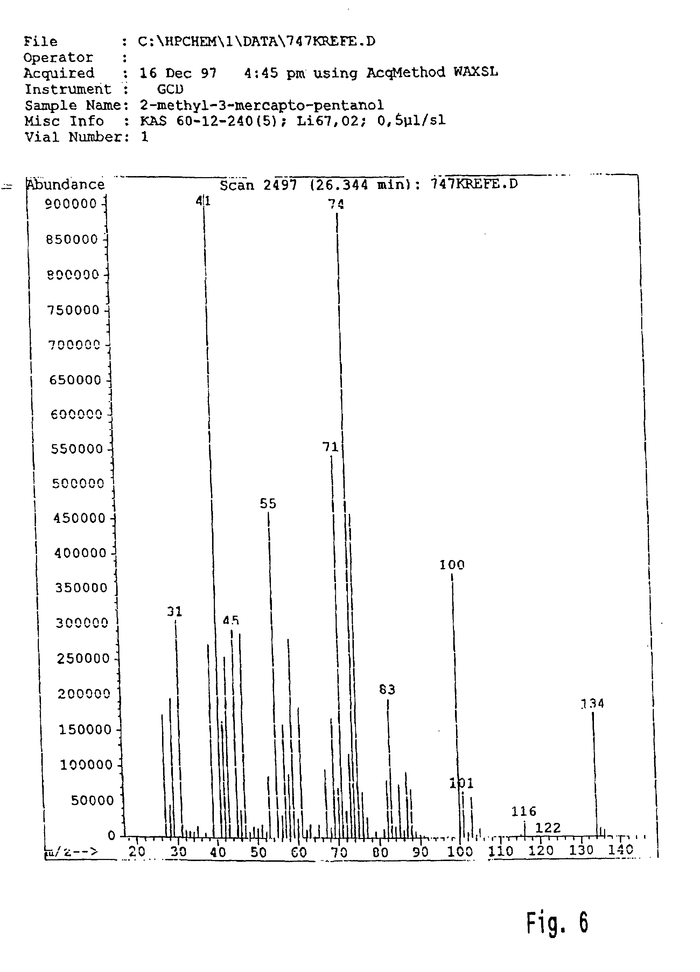

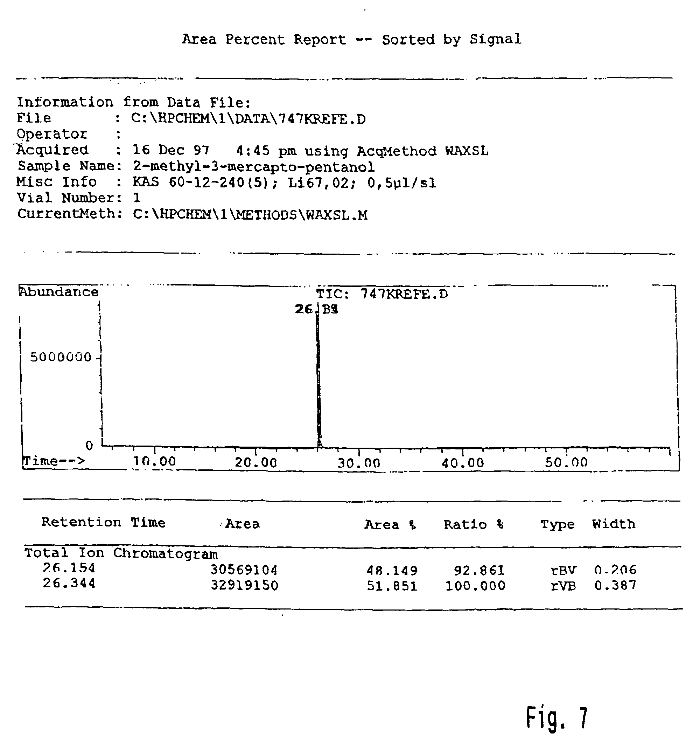

Die zur erfindungsgemäßen Substanz 3-Mercapto-2-methyl-pentan-1-ol angegebenen spektroskopischen Daten entsprechen den beigefügten Spektren gemäß den Figuren 3 - 6; ein Ionenchromatogramm wurde hinzugefügt (Fig. 7).

Soweit die Beispiele 1 und 2.

Die nachfolgenden Beispiele 3 und 4 bilden zusammen eine Herstellvorschrift für die erfindungsgemäße Gruppe der 3-Mercapto-2-alkyl-alkan-1-ole am Beispiel des 3-Mercapto-2-methylpentan-1-ols, wobei in einer ersten Stufe (Beispiel 3) 3-Mercapto-2-methyl-pentanal hergestellt wird.

### Beispiel 3: Herstellung von 3-Mercapto-2-methyl-pentanal

In einem auf 40°C temperierten 500ml-Rührwerk mit Gaseinleitungsrohr werden 321g frisch destilliertes 2-Methyl-2-pentenal, 23g Triethylamin und 1,8g Hydrochinon in 600ml trockenem Tetrahydrofuran gelöst. Bei 40°C wird Schwefelwasserstoff über ca. 6h in einem kräftigen Strom eingeleitet. Der entweichende Schwefelwasserstoff wird durch eine nachgeschaltete Waschflasche mit Natronlauge gebunden. Nach der Reaktion wird für 5min Stickstoff durch die Reaktionslösung geleitet, um überschüssigen Schwefelwasserstoff auszutreiben. Zur Isolierung des 3-Mercapto-2-methyl-pentanals wird die Reaktionslösung mit Wasser und gesättigter Kochsalzlösung gewaschen, über Natriumsulfat getrocknet und im Teilvakuum vom Lösungsmittel befreit.

Bei der GC/MS-Analyse des Rückstandes erhält man beide diastereomeren Mercaptoaldehyde im Verhältnis 1:1 (vgl. auch Beispiel 7).
- Anmerkung:: Das in Beispiel 3 erwähnte 2-Methyl-2-pentenal ist kommerziell erhältlich.
Die sonstigen ungesättigten Aldehyde (2-Alkyl-2-alkenale) zur Synthese analoger 3-Mercapto-2-alkyl-alkanale gemäß Beispiel 3, die ebenfalls umgesetzt wurden aber hier nicht individuell genannt werden, sind entweder ebenfalls kommerziell erhältlich (z.B. 2,3-Dimethylacrolein) oder leicht durch bekannte Syntheseverfahren zugänglich (α,β-ungesättigte Aldehyde durch gezielte Aldolkondensation: L. Brandsma, Preparative Polar Organometallic Chemistry 2, p. 145, Springer Verlag, Heidelberg 1990).

### Beispiel 4: Herstellung von 3-Mercapto-2-methyl-pentan-1-ol (Alternative zur Synthese gemäß Beispiel 2)

Für die Darstellung des Alkohols werden zu der nicht aufgearbeiteten Reaktionslösung aus der ersten Stufe gemäß Beispiel 3 400ml Methanol und 5ml 50%-ige Natronlauge gegeben. Man kühlt mittels Eiskühlung auf 0°C ab und gibt nun in kleinen Portionen 62,4g Natriumborhydrid so zu, daß die Innentemperatur 20°C nicht übersteigt. Nach der Zugabe wird noch 10h bei Raumtemperatur nachgerührt. Anschließend wird das Gemisch am Rotationsverdampfer im Teilvakuum eingeengt und dann mit 800ml Methyl-tert-butylether ausgedünnt. Bei 5-10°C werden zuerst 400ml Wasser und dann 700ml 2 normale Salzzsäure zugegeben. Nach Phasentrennung wird die wäßrige Phase zweimal mit je 200ml Methyl-tert-butylether extrahiert. Die vereinigten organischen Phasen werden einmal mit gesättigter Natriumhydrogencarbonatlösung gewaschen und über Natriumsulfat getrocknet. Nach Abziehen des Lösungsmittels im Teilvakuum wird der Rückstand einer Vakuumdestillation an einer 40cm-Vigreuxkolonne unterworfen. Man erhält 258g des Produkts als klare Flüssigkeit.

### Beispiel 5: Enantiomerenreine Synthese

### 5.1. Synthese der (2R,3S)- und (2S,3R)-Enantiomere des 3-Mercapto-2-methyl-pentan-1-ols

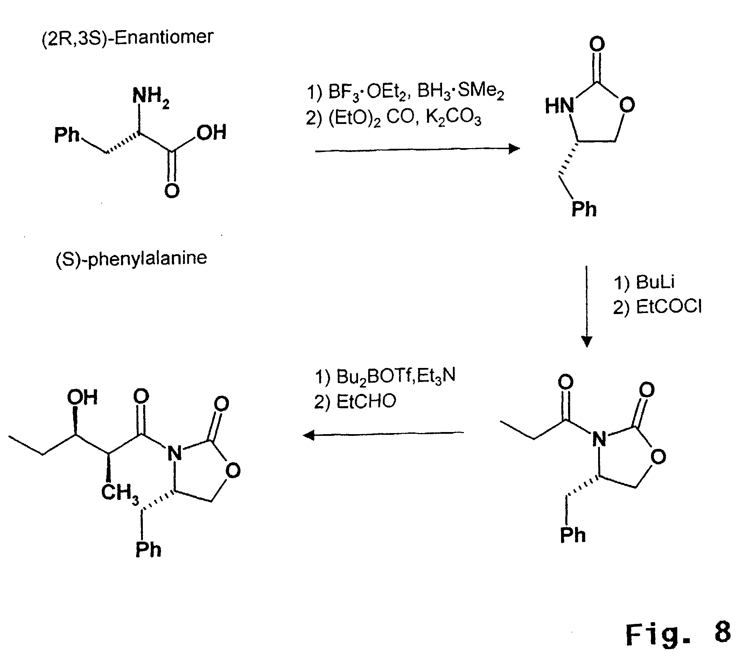

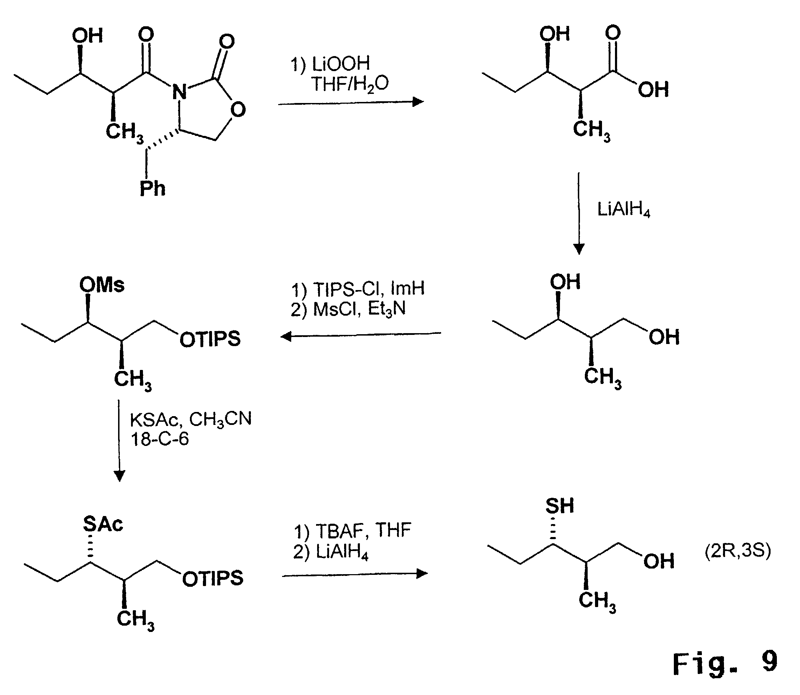

Die in den beigefügten Figuren 8 und 9 schematisch dargestellte Synthese beginnt mit der Reduktion von (S)-Phenylalanin durch Bortrifluorid-Etherat und Boran-Dimethylsulfidkomplex zum Phenylalanol, welches mit Diethylcarbonat zum Oxazolidinon (Evans' Auxiliar) umgesetzt wird. Durch Acylierung des Auxiliars mit Butyllithium und Propionsäurechlorid erhält man das N-Propionyloxazolidinon. In einer diastereoselektiven Aldolreaktion mit Dibutylbortrifluormethansulfonat, Triethylamin und Propionaldehyd läßt sich, nach Umkristallisation, das diastereomerenreine (S)-3-[(2S,3R)-3-hydroxy-2-methyl-1-oxopentyl]-4-phenylmethyl-1,3-oxazolidin-2-on gewinnen (Figur 8). Durch selektive Spaltung des Acylrestes vom Oxazolidinon mittels Lithiumhydroperoxid erhält man daraus die enantiomerenreine ß-Hydroxysäure, die durch Lithiumaluminiumhydrid-Reduktion das 1,3-Diol ergibt (J. Michael Chong, Tetrahedron 1994, 50, 2703 und Referenzen darin). Nach selektiver Schützung der primären Hydroxylgruppe als Triisopropylsilylether erzeugt man durch Reaktion der sekundären Hydroxylgruppe mit Methansulfonsäurechlorid eine Fluchtgruppe, die in der anschließenden Reaktion mit Kaliumthioacetat, 18-Krone-6 in Acetonitril unter Inversion der Konfiguration substituiert wird. Entschützung der primären Hydroxylgruppe und Lithiumaluminiumhydrid-Reduktion des Thioesters liefert das enantiomerenreine (2R,3S)-3-Mercapto-2-methyl-pentan-1-ol (Figur 9).

Die Darstellung des (2S,3R)-Enantiomers erfolgt, ausgehend von (R)-Phenyl-alanin, entsprechend.

### 5.2. Synthese der (2R,3R)- und (2S,3S)-Enantiomere des 3-Mercapto-2-methyl-pentan-1-ols

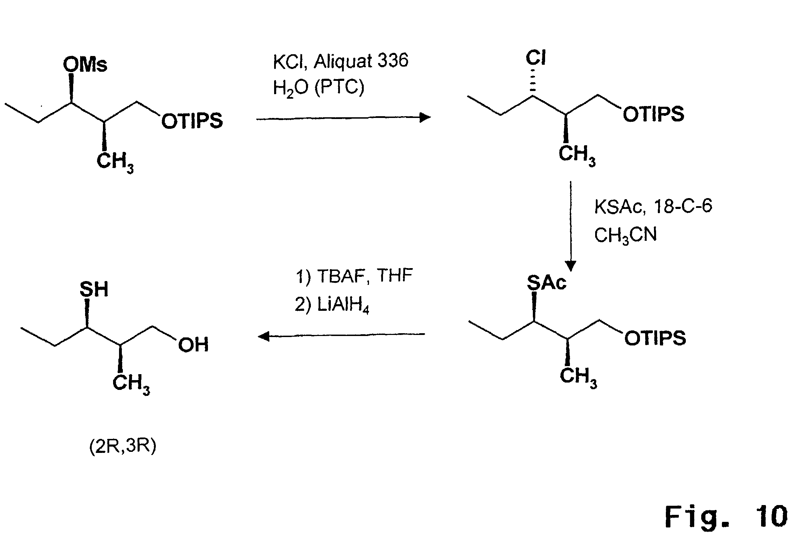

Durch Umsetzung der Methansulfonate aus den vorhergehenden Synthesen (siehe 5.1) erhält man mit Kaliumchlorid, Aliquat 336 und Wasser unter Phasentransferbedingungen die Chloride mit umgekehrter Konfiguration. Aus diesen lassen sich durch erneute Substitutionsreaktionen mit Kaliumthioacetat die syn-Thioacetate darstellen, die nach Entschützung und Reduktion des Thioesters die entsprechenden 3-Mercapto-2-methyl-pentanole ergeben (Figur 10).

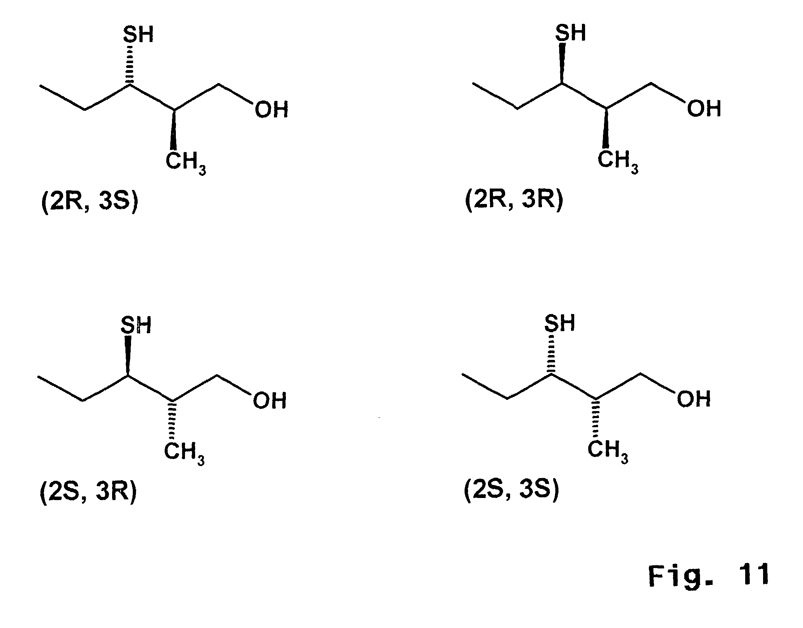

In Fig. 11 sind die vier gemäß Beispiel 5 synthetisierten Enantiomere des 3-Mercapto-2-methyl-pentanol als Beispiel für die verschiedenen Enantiomeren der erfindungsgemäßen Alkanole nebeneinander dargestellt.

### Beispiel 6: Sensorische Untersuchung ausgewählter erfindungsgemäßer Verbindungen:

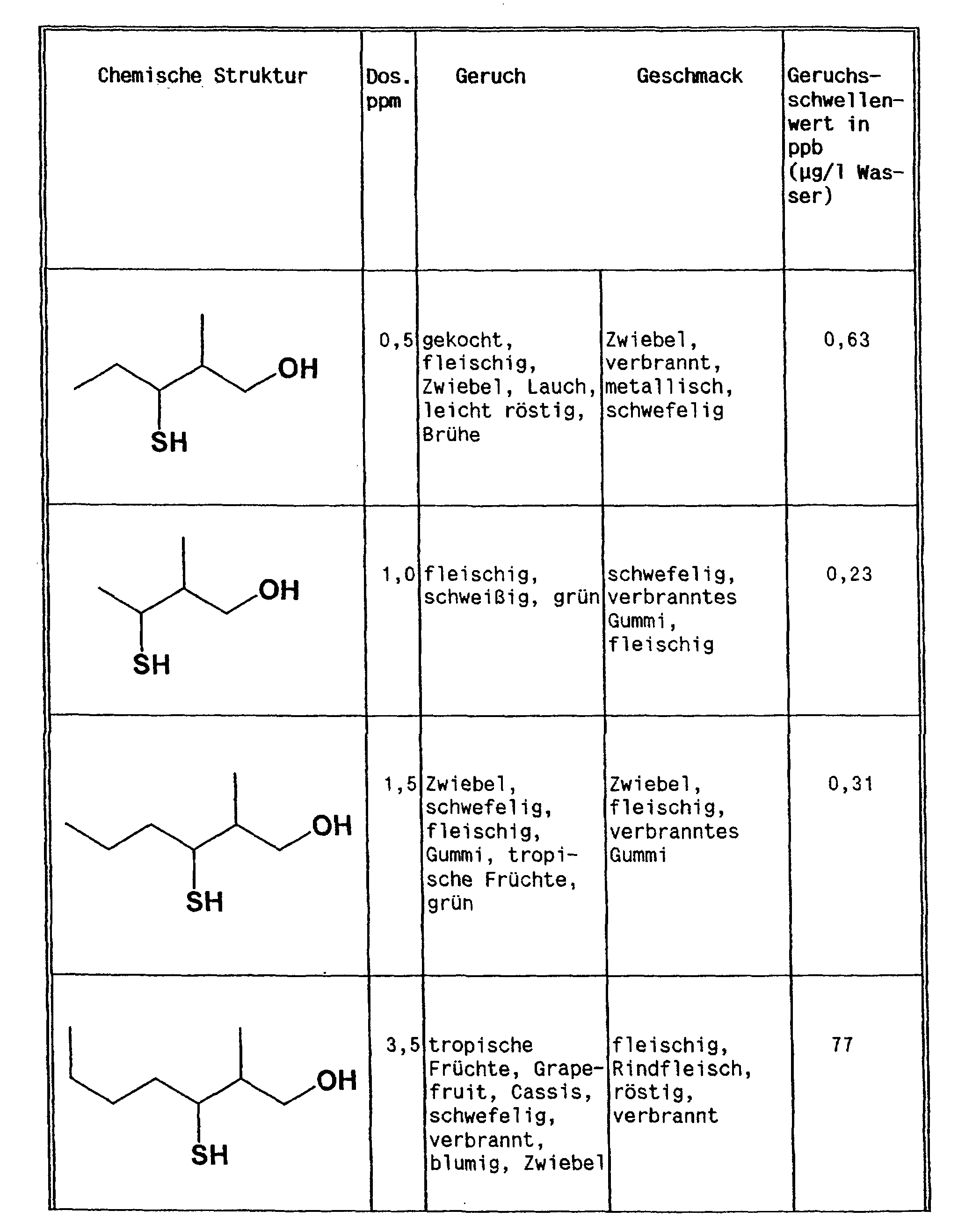

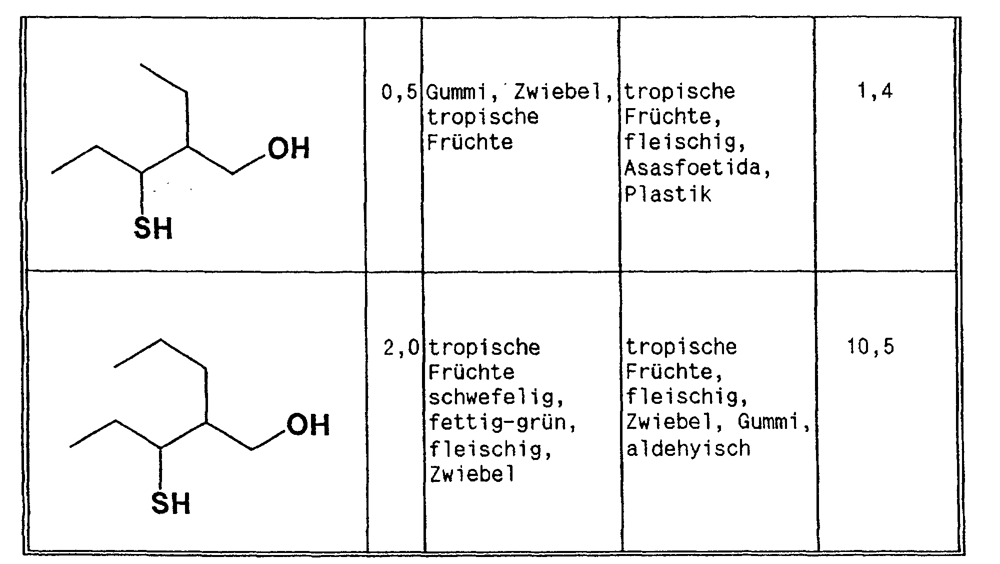

Für 3(S)-Mercapto-2(R)-methyl-pentan-1-ol wurden die Geruchsschwellen in Wasser und Luft bestimmt. Ergebnis:
- Wasser:: 0,09 ppb (µg/L)
- Luft:: 0,00007-0,00014 ng/L Luft

Die Geruchsschwelle in Luft zählt zu den niedrigsten Geruchsschwellen, die jemals für Aromastoffe bestimmt wurden.

### Beispiel 7: Spektroskopische Daten weiterer ausgewählter Verbindungen

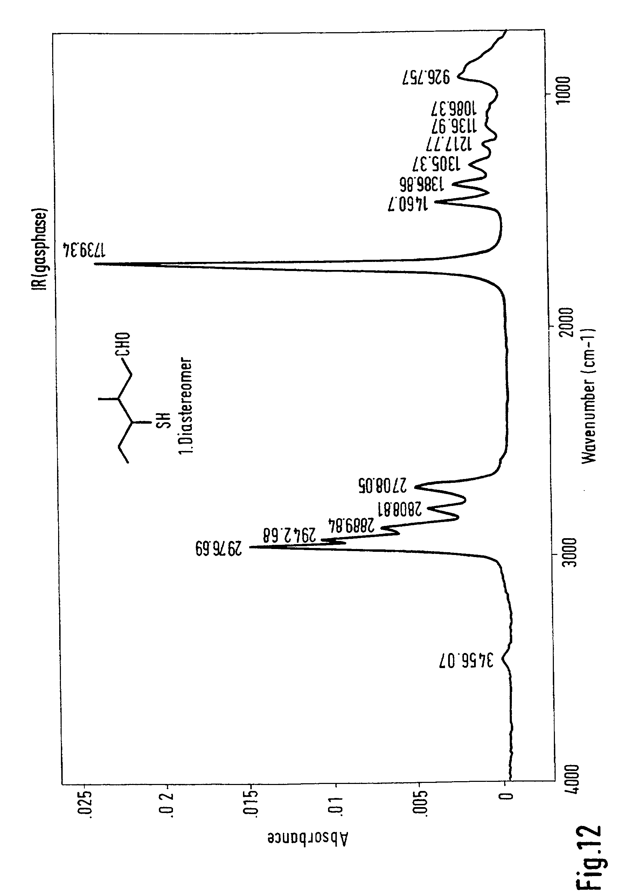

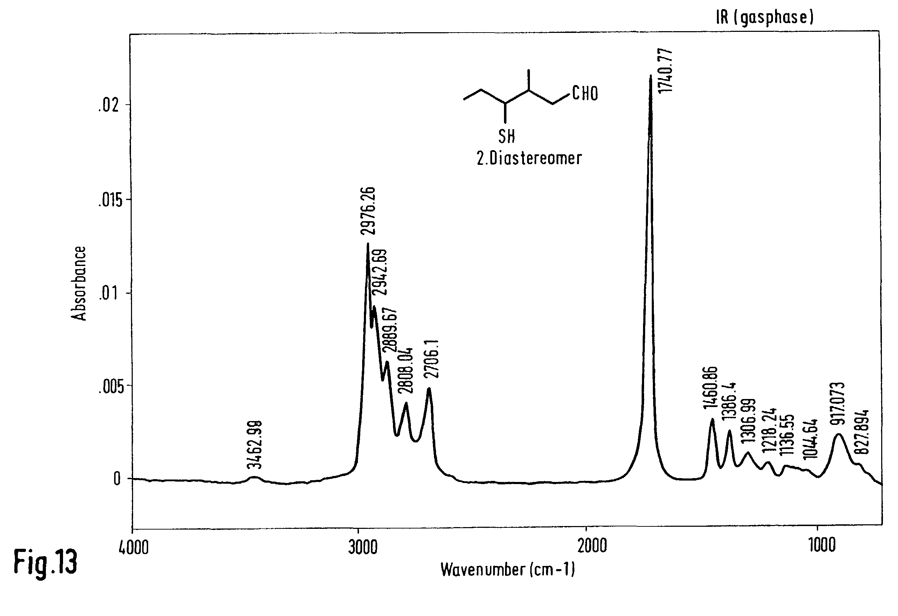

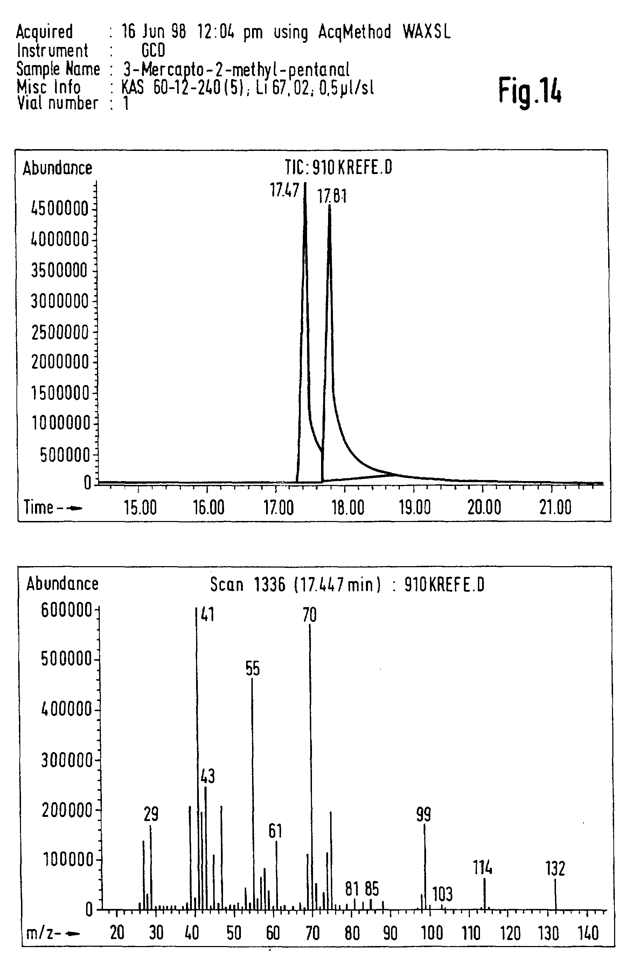

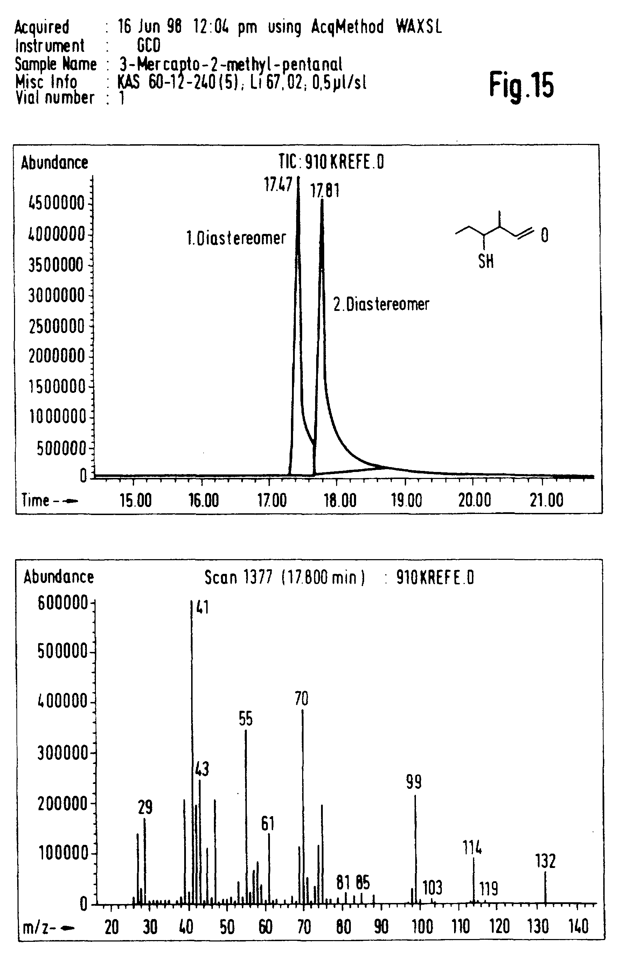

3-Mercapto -2-methyl-pentanal (siehe Beispiele 3 und 4)
   **FTIR** (gas-phase): Diastereomer 1: 2977 (m), 2942 (m), 2890 (m), 2809 (w), 2708 (m), 1739 (s), 1460 (w), 1387 (w), 1305 (w); Diastereomer 2: 2976 (m), 2942 (m), 2890 (m), 2808 (w), 2706 (m), 1740 (s), 1461 (w), 1386 (w), 1307 (w)
   w=schwache, m=mittelstarke, s=starke Bande
   Diesen Daten entsprechen die Spektren gemäß den Figuren 12 und 13
   **MS** (EI, 70 eV): Diastereomer 1: 132 (M⁺, 12), 114 (13), 99 (30), 75 (33), 70 (100), 61 (24), 55 (82), 43 (39), 41 (97); Diastereomer 2: 132 (M⁺, 13) 114 (18); 99 (41), 75 (39), 70 (75), 61 (24), 55 (67), 43 (43), 41 (100)
   Diesen Daten entsprechen die Spektren gemäß den Figuren 14 und 15
3-Mercapto-2-methyl-butan-1-ol
   **MS** (EI, 70 eV): 120 (M⁺, 31), 102 (4), 86 (84), 71 (77), 69 (38), 61 (89), 60 (100), 55 (58), 45 (67), 41 (69), 31 (42)
3-Mercapto-2-methyl-hexan-1-ol
   **MS** (EI, 70 eV): 148 (M⁺, 11), 130 (1), 114 (21), 97 (10), 88 (37), 71 (37), 55 (100), 47 (23), 41 (37)
3-Mercapto-2-methyl-heptan-1-ol
   **MS** (EI, 70 eV): 162 (M⁺, 10), 128 (18), 102 (17), 97 (11), 87 (34), 71 (49), 69 (100), 60 (62), 55 (60), 41 (58)
2-Ethyl-3-mercapto-pentan-1-ol
   **MS** (EI, 70 eV): 148 (M^{+,} 23), 130 (8), 114 (62), 85 (70), 74 (100), 55 (97), 41 (82)
2-Propyl-3-mercapto-pentan-1-ol
   **MS** (EI, 70 eV): 162 (M⁺, 21), 144 (6), 128 (45), 74 (100), 55 (72)

## Patentansprüche

1. 3-Mercapto-2-alkyl-alkan-1-ol der allgemeinen Formel A, 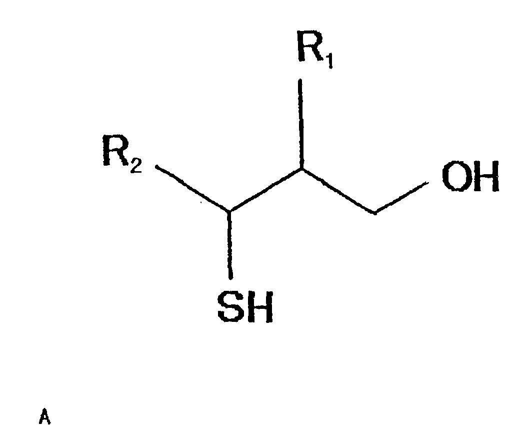 mit
R₁ = CH₃, ·C₂H₅, C₃H₇ (n, iso) oder C₄H₉ (n, iso, tert.) und
R₂ = CH₃, C₂H₅, C₃H₇ (n, iso) oder C₄H₉ (n, iso, tert.).

2. Verfahren zur Herstellung eines 3-Mercapto-2-alkyl-alkan-1-ols gemäß Anspruch 1, in dem das korrespondierende 3-Acetylthio-2-alkyl-alkanal mit einem Reduktionsmittel wie Lithiumaluminiumhydrid oder Diisobutylaluminiumhydrid umgesetzt wird.

3. Verfahren zur Herstellung von 3-Mercapto-2-methyl-pentan-1-ol, in dem 3-Acetylthio-2-methyl-pentanal mit einem Reduktionsmittel wie Natriumborhydrid, Lithiumaluminiumhydrid oder Diisobutylaluminiumhydrid umgesetzt wird.

4. Verfahren zur Herstellung eines 3-Mercapto-2-alkyl-alkan-1-ols gemäß Anspruch 1, in dem das korrespondierende 3-Mercapto-2-Alkyl-Alkanal mit einem Reduktionsmittel wie Natriumborhydrid, Lithiumaluminiumhydrid oder Diisobutylaluminiumhydrid umgesetzt wird.

5. Verwendung einer Verbindung gemäß Anspruch 1 als Duft- oder Aromastoff.

6. Verwendung von 3-Hercapto-2-methyl-pentan-1-ol als Duft- oder Aromastoff.

7. Duft- oder Aromastofformulierung, umfassend zumindest eine Verbindung gemäß Anspruch 1.

8. Aromatisiertes Lebensmittel mit einem Anteil an einer Verbindung gemäß Anspruch 1.

9. Aromatisiertes Lebensmittel mit einem Anteil an 3-Mercapto-2-methyl-pentan-1-ol.

10. Verfahren zur Aromatisierung eines Lebensmittels mit einem Alkanol gemäß Anspruch 1, wobei
- das Lebensmittel mit einem Ester des Alkanols versetzt
- und das mit dem Ester versetzte Lebensmittel einer Behandlung unterzogen wird, bei der der Ester zumindest partiell in das Alkanol überführt wird.

## Claims

1. 3-Mercapto-2-alkyl-alkan-1-ol of the general formula A, 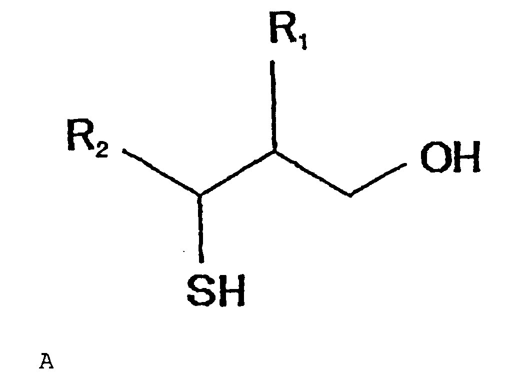 with
R₁ = CH₃, C₂H₅, C₃H₇ (n, iso) or C₄H₉ (n, iso, tert.) and
R₂ = CH₃, C₂H₅, C₃H₇ (n, iso) or C₄H₉ (n, iso, tert.).

2. Process for the production of a 3-mercapto-2-alkyl-alkan-1-ol according to claim 1, in which the corresponding 3-acetylthio-2-alkyl-alkanal is reacted with a reducing agent such as lithium aluminium hydride or diisobutylaluminium hydride.

3. Process for the production of 3-mercapto-2-methyl-pentan-1-ol, in which 3-acetylthio-2-methyl-pentanal is reacted with a reducing agent such as sodium borohydride, lithium aluminium hydride or diisobutylaluminium hydride.

4. Process for the production of a 3-mercapto-2-alkyl-alkan-1-ol according to claim 1, in which the corresponding 3-mercapto-2-alkyl-alkanal is reacted with a reducing agent such as sodium borohydride, lithium aluminium hydride or diisobutylaluminium hydride.

5. Use of a compound according to claim 1 as a perfuming or flavouring agent.

6. Use of 3-mercapto-2-methyl-pentan-1-ol as a perfuming or flavouring agent.

7. Perfuming or flavouring agent formulation comprising at least one compound according to claim 1.

8. Flavoured foodstuff with a proportion of a compound according to claim 1.

9. Flavoured foodstuff with a proportion of 3-mercapto-2-methyl-pentan-1-ol.

10. Process for the flavouring of a foodstuff with an alkanol according to claim 1, wherein
- an ester of the alkanol is added to the foodstuff
- and the foodstuff to which the ester has been added is subject to a treatment in which the ester is at least partially converted to the alkanol.

## Revendications

1. 3-mercapto-2-alkyl-alcan-ol selon la formule générale A, 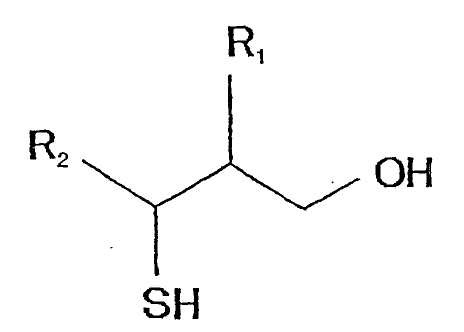 où
R₁ = CH₃, C₂H₅, C₃H₇ (n, iso) ou C₄H₉ (n, iso, tert) et
R₂ = CH₃, C₂H₅, C₃H₇ (n, iso) ou C₄H₉ (n, iso, tert).

2. Procédé pour la fabrication d'un 3-mercapto-2-alkyl-alcan-1-ol selon la revendication 1, dans lequel le 3-acétylthio-2-alkyl-alcanal correspondant est décomposé à l'aide d'un réducteur tel que l'hydrure de lithium-aluminium ou l'hydrure de diisobutylaluminium.

3. Procédé pour la fabrication d'un 3-mercapto-2-méthyl-pentan-1-ol dans lequel le 3-acétylthio-2-méthyl-pentanal est décomposé à l'aide d'un réducteur tel que le borohydrure de sodium, l'hydrure de lithium-aluminium ou l'hydrure de diisobutylaluminium.

4. Procédé pour la fabrication d'un 3-mercapto-2-alkyl-alcan-1-ol selon la revendication 1, dans lequel le 3-mercapto-2-alkyl-alcanal correspondant est décomposé à l'aide d'un réducteur tel que le borohydrure de sodium, l'hydrure de lithium-aluminium ou l'hydrure de diisobutylaluminium.

5. Utilisation d'un composé selon la revendication 1 comme agent parfumant ou aromatisant.

6. Utilisation de 3-mercapto-2-méthyl-pentan-1-ol comme agent parfumant ou aromatisant.

7. Formulation d'agents parfumants ou aromatisants comprenant au moins un composé selon la revendication 1

8. Aliment aromatisé avec une portion d'un composé selon la revendication 1.

9. Aliment aromatisé avec une portion de 3-mercapto-2-méthyl-pentan-1-ol.

10. Procédé pour l'aromatisation d'un aliment avec un alcanol selon la revendication 1, dans lequel
- l'aliment est mélangé à un ester de l'alcanol
- et l'aliment mélangé à l'ester est soumis à un traitement au cours duquel l'ester est transféré au moins partiellement dans l'alcanol.
